# EUROPEAN PATENT APPLICATION

(11) **EP 2 679 164 A1**
(43) Date of publication of application: **01.01.2014**
(21) Application number: 13171747.2
(22) Date of filing: 12.06.2013
(51) Int. Cl.: A61B 8/06, A61B 8/00, A61B 8/08

(54) **Ultrasonic diagnosis apparatus and control program thereof**

(30) Priority: 28.06.2012 JP 2012145905
(71) Applicant: GE Medical Systems Global Technology Company LLC, Waukesha, Wisconsin 53188-1696 (US)
(72) Inventor: Hashimoto, Hiroshi, Hino-shi Tokyo, 191-8503 (JP); Sato, Naoto, Hino-shi Tokyo, 191-8503 (JP); Miyami, Koji, Hino-shi Tokyo, 191-8503 (JP); Kato, Sei, Hino-shi Tokyo, 191-8503 (JP)
(74) Representative: Bedford, Grant Richard

(57) **Abstract**

An ultrasonic diagnostic apparatus 1 capable of displaying an image having considered two or more parameters of parameters each indicative of a state of inflow of a contrast agent and parameters each indicative of outflow of the contrast agent is provided. The ultrasonic diagnostic apparatus 1 is equipped with a display controller which causes a parametric image generated using a two-dimensional map MP1 taking as axes, an arrival time T1 taken until a contrast agent arrives and an outflow time T2 taken until the contrast agent flows out, for example, of parameters each indicative of a state of inflow of the contrast agent administered to a subject and parameters each indicative of a state of outflow of the contrast agent, to be displayed based on the arrival time T1 and the outflow time T2. The parameter indicative of the state of inflow of the contrast agent may be an inflow rate of the contrast agent at a portion at which the contrast agent has arrived.

## Description

The present invention relates to an ultrasonic diagnostic apparatus which allows an image of a subject administered with a contrast agent to be displayed, and a control program thereof.

There has been known an ultrasonic diagnostic apparatus which performs transmission/reception of ultrasound to and from a subject administered with a contrast agent and allows a contrast image emphasized in contrast agent to be displayed based on echo signals obtained by such transmission/reception. In the contrast image, a portion desired to observe using the contrast agent and a portion other than the portion can be displayed on the image with a positive difference made therebetween. See, for example, Japanese Unexamined Patent Publication No. 2012-24132.

Meanwhile, there is a case in which upon a contrast examination of a liver, for example, a diagnosis thereof is performed based on a plurality of parameters such as a time taken until a contrast agent arrives, a rate (velocity) of a rise in the brightness of a contrast image from the arrival of the contrast agent, a time taken until the contrast agent flows out since its arrival, etc. Thus, the ultrasonic diagnostic apparatus is accompanied by a problem in displaying an image having taken into consideration two or more parameters of parameters each indicative of a state of inflow of a contrast agent and parameters each indicative of outflow of the contrast agent.

One aspect of the invention made to address the above problem is an ultrasonic diagnostic apparatus equipped with a display controller which causes an image generated using a map taking as axes, two or more parameters of parameters each indicative of a state of inflow of a contrast agent administered to a subject and parameters each indicative of a state of outflow of the contrast agent to be displayed based on the two or more parameters.

According to the invention of the above aspect, an image generated using a map taking as axes, two or more parameters of parameters each indicative of a state of inflow of a contrast agent and parameters each indicative of a state of outflow of the contrast agent is displayed. It is therefore possible to display an image that takes into consideration two or more parameters.

Various aspects and embodiments of the present invention will now be described in connection with the accompanying drawings, in which:
FIG. 1 is a block diagram showing one example of a schematic configuration of an embodiment of an ultrasonic diagnostic apparatus according to an embodiment of the present invention.
FIG. 2 is a block diagram illustrating a configuration of an echo data processor in the ultrasonic diagnostic apparatus shown in FIG. 1.
FIG. 3 is a diagram depicting one example of a two-dimensional map in a first embodiment.
FIG. 4 is a diagram showing a brightness change curve related to a contrast image.
FIG. 5 is a diagram illustrating a displayer on which a parametric image, a contrast image and a B-mode image are displayed.
FIG. 6 is a diagram depicting a region in which a parametric image is displayed at a subj ect.
FIG. 7 is a diagram showing a two-dimensional map illustrative of points corresponding to the region shown in FIG. 6.
FIG. 8 is a diagram illustrating one example of a parametric image.
FIG. 9 is a diagram showing one example of a two-dimensional map in a second embodiment.
FIG. 10 is a diagram illustrating a brightness change curve related to a contrast image.
FIG. 11 is a diagram depicting a brightness change curve related to a contrast image.
FIG. 12 is a diagram showing one example of a two-dimensional map in a third embodiment.
FIG. 13 is a diagram illustrating one example of a three-dimensional map in a fourth embodiment.

Various embodiments of the present invention will hereinafter be described.

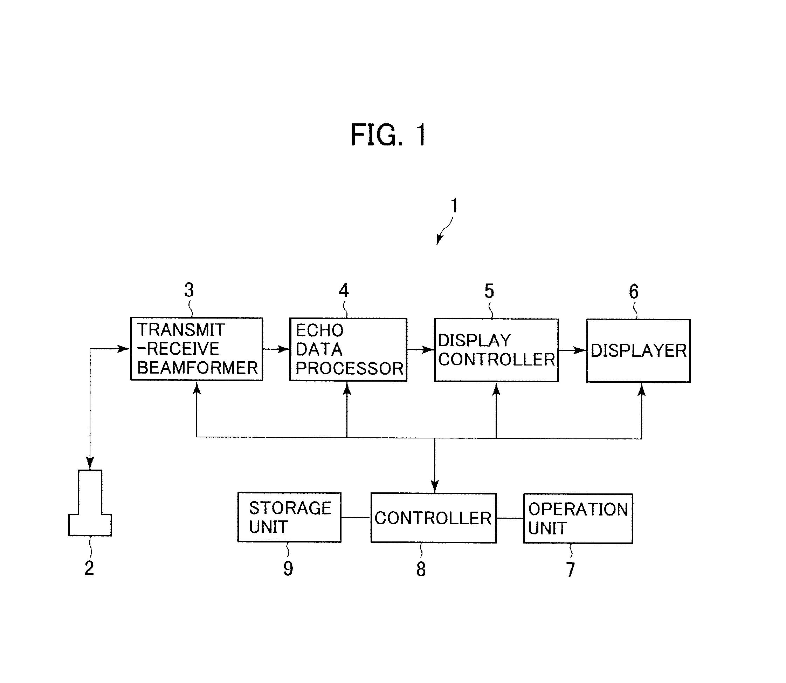

A first embodiment will first be explained based on FIGS. 1 through 8. An ultrasonic diagnostic apparatus 1 shown in FIG. 1 is equipped with an ultrasonic probe 2, a transmit-receive beamformer 3, an echo data processor 4, a display controller 5, a displayer 6, an operation unit 7, a controller 8 and a storage unit 9.

The ultrasonic probe 2 is comprised of a plurality of ultrasonic transducers (not shown) arranged in array form. The ultrasonic probe 2 transmits ultrasound to a subject through the ultrasonic transducers and receives its echo signals.

The transmit-receive beamformer 3 supplies an electric signal for transmitting ultrasound from the ultrasonic probe 2 under a predetermined scan condition to the ultrasonic probe 2, based on a control signal outputted from the controller 8. The transmit-receive beamformer 3 performs signal processing such as A/D conversion, phasing-adding processing and the like on each echo signal received by the ultrasonic probe 2 and outputs echo data subsequent to the signal processing to the echo data processor 4.

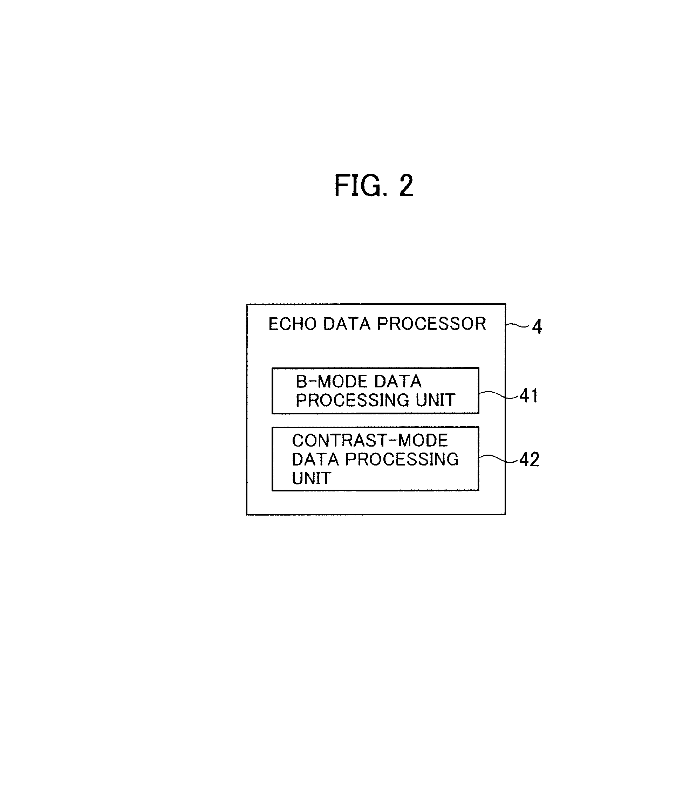

The echo data processor 4 has a B-mode data processing unit 41 and a contrast-mode data processing unit 42 as shown in FIG. 2. The B-mode data processor 4 performs B-mode processing including logarithmic compression processing and envelop detection processing to generate B-mode data.

The contrast-mode data processing unit 42 performs processing for generating a contrast image in which a contrast agent administered to the subject is emphasized, on the echo data outputted from the transmit-receive beamformer 3 to generate contrast-mode data. For example, the contrast-mode data processing unit 42 performs filter processing for extracting harmonic components of each echo signal. The contrast-mode data processing unit 42 may perform processing for extracting each echo signal from the contrast agent by a Pulse Inversion method. Alternatively, the contrast-mode data processing unit 42 may perform processing (Amplitude Modulation) that performs a subtraction of echo data based on echo signals obtained by transmitting ultrasound different in amplitude to extract signal components from the contrast agent. The contrast-mode data processing unit 42 is one example illustrative of an embodiment of a contrast-mode signal processing unit in the present invention.

The display controller 5 performs scan conversion based on a scan converter on the B-mode data and the contrast-mode data to generate B-mode image data and contrast image data. Incidentally, the pre-scan conversion B-mode data and contrast-mode data will be called raw data.

The display controller 5 causes the displayer 6 to display a B-mode image based on the B-mode image data and causes the displayer 6 to display a contrast image based on the contrast-mode data. Further, based on two or more parameters of parameters each indicative of a state of inflow of a contrast agent administered to the subject, and parameters each indicative of a state of outflow of the contrast agent, the display controller 5 generates a parametric image generated using a map MP with the two or more parameters as axes and allows the displayer 6 to display it (display control function). The map MP is a map in which display forms (hue, brightness, chroma, etc.) are allocated according to the respective parameters. The details thereof will be described later. The display controller 5 is one example illustrative of an embodiment of a display controller in the present invention.

The displayer 6 is an LCD (Liquid Crystal Display), a CRT (Cathode Ray Tube) or the like. The operation unit 7 includes a keyboard and a pointing device (not shown) or the like for inputting instructions and information by an operator.

The controller 8 is a CPU (Central Processing Unit) and reads a control program stored in the storage unit 9 to execute functions at the respective parts of the ultrasonic diagnostic apparatus 1, starting with the display control function.

The storage unit 9 is, for example, an HDD (Hard Disk Drive), a semiconductor memory or the like.

A description will now be made of the operation of the ultrasonic diagnostic apparatus 1 according to the present embodiment. The ultrasonic diagnostic apparatus 1 displays the parametric image. Data for generating a parametric image is first acquired to display the parametric image. Specifically, the transmission/reception of ultrasound is first performed on the subject administered with the contrast agent by the ultrasonic probe 2. The B-mode data processing unit 41 generates B-mode data, based on acquired echo signals. The contrast-mode data processing unit 42 generates contrast-mode data, based on the acquired echo signals. The contrast-mode data is data for generating the parametric image. The contrast-mode data is stored in the storing unit 9. The B-mode data is also stored in the storage unit 9.

Upon the transmission/reception of ultrasound, real-time B-mode and contrast images may be displayed. Specifically, the display controller 5 generates B-mode image data, based on the B-mode data and generates contrast image data, based on the contrast-mode data. And the display controller 5 allows the displayer 6 to display a B-mode image based on the B-mode image data and a contrast image based on the contrast image data in real time. The B-mode image and the contrast image may be displayed in overlay form or displayed side by side.

The B-mode image data and the contrast image data may be stored in the storage unit 9. In this case, the parametric image may be generated based on the contrast image data.

When the contrast-mode data is acquired, the display controller 5 causes the displayer 6 to display the parametric image. This parametric image may be displayed after the completion of the display of a real-time image or may be displayed along with the real-time image.

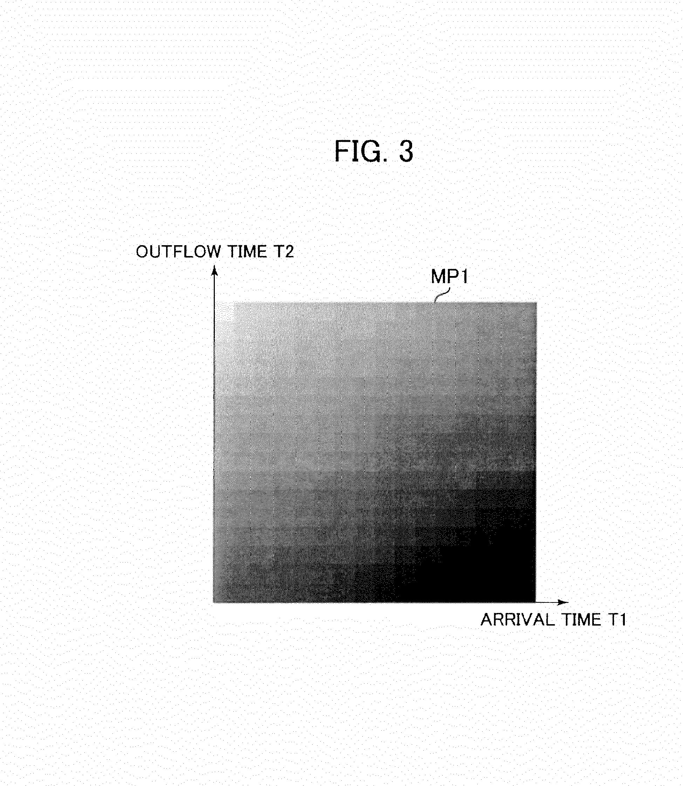

A description will be made of the generation of the parametric image. The display controller 5 generates and displays a parametric image using a two-dimensional map MP with two of parameters each indicative of a state of inflow of a contrast agent administered to a subject and parameters each indicative of a state of outflow of the contrast agent being taken as the axes. In the present embodiment, as shown in FIG. 3, the parametric image is generated using a two-dimensional map MP1 in which the horizontal axis indicates an arrival time T1 of the contrast agent and the vertical axis indicates an outflow time T2 up to the outflow of the contrast agent. This two-dimensional map MP1 is a color map and a map in which colors corresponding to the respective parameter values of the arrival time T1 and the outflow time T2 are allocated.

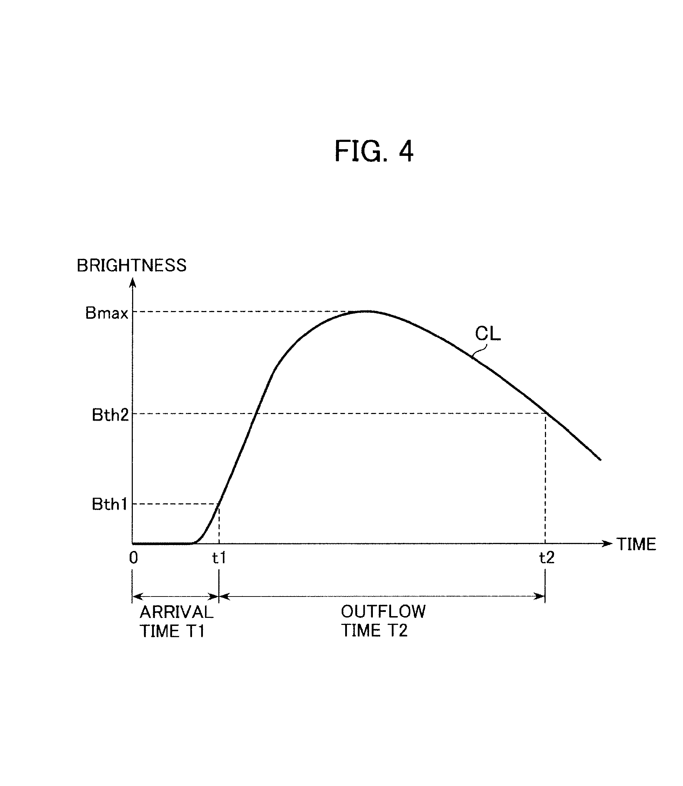

The arrival time T1 and the outflow time T2 will be explained based on FIG. 4. A brightness change curve CL at a given pixel is shown in FIG. 4. The arrival time T1 indicates a time at which the contrast agent arrives with a predetermined time as a starting point. In the present embodiment, if the time at which an input to notify the administration of the contrast agent is performed at the operation unit 7, is assumed to be zero, then the arrival time T1 corresponds to a time from this time to a time t1 at which the brightness of the contrast agent has reached a first threshold value Bth1. Thus, the display controller 5 measures the time from the time when the input is done to the time t1 when the value of contrast-mode data reaches a data value corresponding to the first threshold value Bth1, based on the contrast-mode data. The display controller 5 may, however, perform the measurement of the arrival time T1, based on the contrast image data without using the contrast-mode data. The arrival time T1 is one example illustrative of an embodiment of a parameter indicative of a state of inflow of the contrast agent.

Incidentally, the input made to notify the administration of the contrast agent at the operation unit 7 may be an input made to bring a contrast clock to on, for example.

The outflow time T2 is a time taken until the contrast agent flows out from a region or portion in which the contrast agent flows with a predetermined time as a starting point. In the present embodiment, the outflow time T2 corresponds to the time from the times t1 to t2. The time t2 corresponds to a time at which the brightness of a contrast image falls below a second threshold value Bth2 (Bth1<Bth2<Bmax) after it has reached the maximum brightness value Bmax. The display controller 5 measures, based on the contrast-mode data, a time from the time t1 to the time t2 at which the value of the contrast-mode data falls below a data value corresponding to the second threshold value Bth2. The display controller 5 may, however, perform the measurement of the outflow time T2, based on the contrast image data without using the contrast-mode data. The outflow time T2 is one example illustrative of an embodiment of a parameter indicative of a state of outflow of the contrast agent.

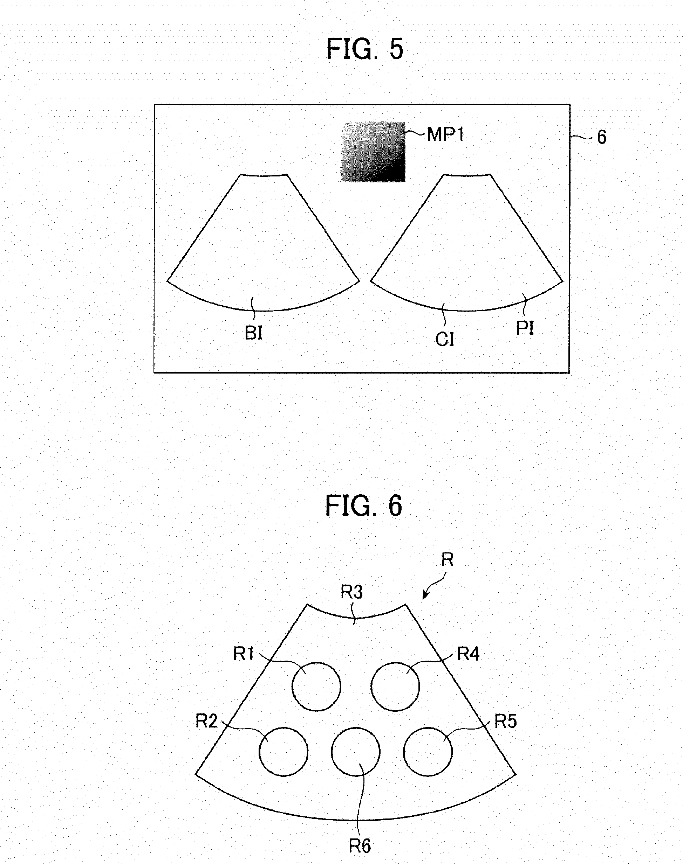

The display controller 5 specifies the arrival time T1 and the outflow time T2, based on the contrast-mode data. The display controller 5 generates a color parametric image using the two-dimensional map and displays it on the displayer 6. As shown in FIG. 5, a parametric image PI may be displayed on a contrast image CI at the displayer 6. A B-mode image BI may also be displayed on the displayer 6 side by side with the parametric image PI and the contrast image CI. The two-dimensional map MP1 may be displayed on the displayer 6.

The contrast image CI is an image generated based on the contrast-mode data stored in the storage unit 9. The B-mode image BI is an image generated based on the B-mode data stored in the storage unit 9. When, however, contrast image data and B-mode image data are being stored therein, images (contrast image CI and B-mode image BI) based on these contrast image data and B-mode image data may directly be displayed.

The parametric image PI will be explained in detail. As shown in FIG. 6, a region of a subject in which a parametric image PI is displayed, is assumed to be R. In this region R, regions R1, R2, R3, R4, R5 and R6 are respectively regions having the following characteristics:
R1: region in which after the brightness of a contrast image has exceeded the first threshold value Bth1 relatively early, it falls below the second threshold value Bth2 relatively early (region in which the contrast agent arrives earlier and its outflow is also fast),
R2: region in which after the brightness of the contrast image has exceeded the first threshold value Bth1 relatively slowly, it falls below the second threshold value Bth2 relatively slowly (region in which the arrival of the contrast agent is slow and its outflow is also slow),
R3: region in which after the brightness of the contrast image has exceeded the first threshold value Bth1, it does not fall below the second threshold value Bth2 within a predetermined time (t2max to be described later) (region in which the contrast agent resists becoming its outflow),
R4: region in which after the brightness of the contrast image has exceeded the first threshold value Bth1 relatively early, it does not fall below the second threshold value Bth2 within the predetermined time (t2max to be described later) (region in which the arrival of the contrast agent is fast and the contrast agent resists becoming its outflow),
R5: region in which after the brightness of the contrast image has exceeded the first threshold value Bth1 relatively slowly, it does not fall below the second threshold value Bth2 within the predetermined time (t2max to be described later) (region in which the arrival of the contrast agent is slow and the contrast agent resists becoming it outflow), and
R6: region in which the brightness of the contrast image does not exceed the first threshold value Bth1 within a predetermined time (t1max to be described later) (region in which the contrast agent does not reach).

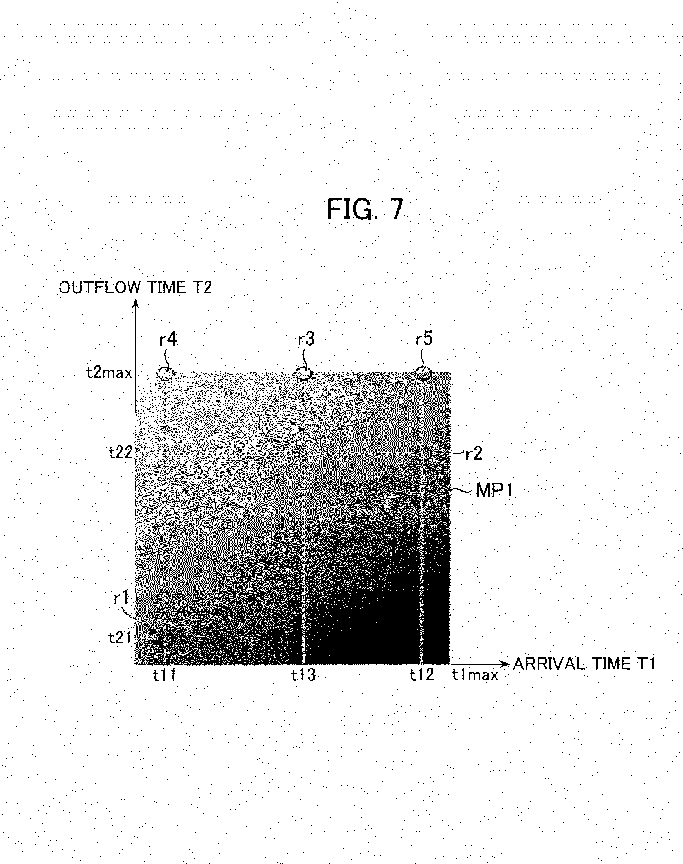

As shown in FIG. 7, in the two-dimensional map MP1, a point corresponding to the region R1, a point corresponding to the region R2, a point corresponding to the region R3, a point corresponding to the region R4 and a point corresponding to the region R5 are respectively assumed to be r1, r2, r3, r4 and r5. Incidentally, the colors of the two-dimensional map MP1 have been allocated till the times t1max and t2max. When the brightness of the contrast image does not exceed the first threshold value Bth1 before the time t1max, the parametric image PI is assumed not to be colored. When the brightness of the contrast image does not fall below the second threshold value Bth2 before the time t2max after it has exceeded the first threshold value Bth1, the parametric image PI is generated assuming the time t2max to be an outflow time T2.

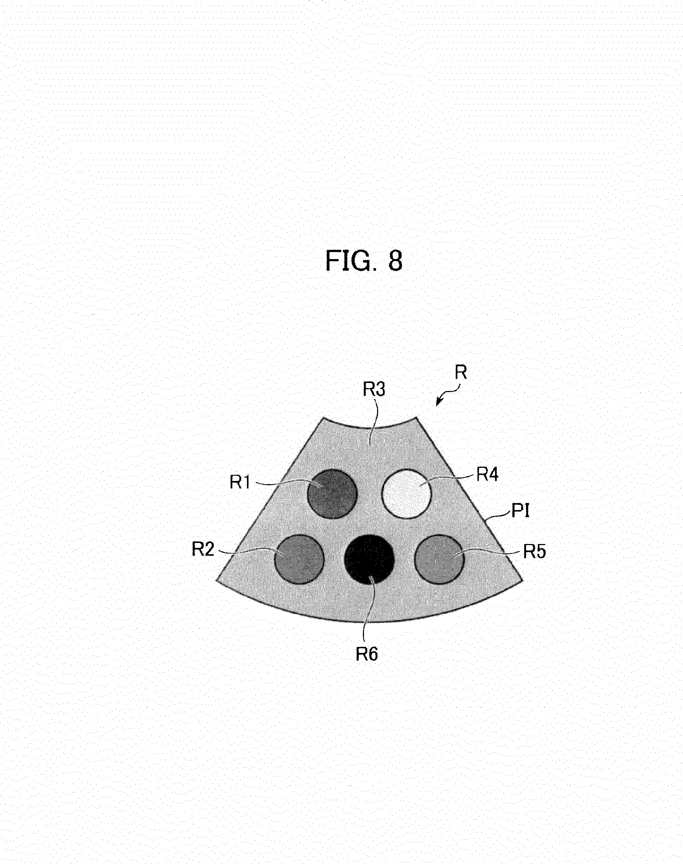

One example of the parametric image PI displayed on the displayer 6 is shown in FIG. 8. In FIG. 8, only the parametric image PI is shown in enlarged form. In the parametric image PI, a color corresponding to the point r1 at which the arrival time T1 is t11 and the outflow time T2 is t21 is displayed in the region R1. A color corresponding to the point r2 at which the arrival time T1 is t12 and the outflow time T2 is t22 is displayed in the region R2. Likewise, in the parametric image PI, a color corresponding to the point r3 at which the arrival time T1 is t13 and the outflow time T2 is t2max is displayed in the region R3. A color corresponding to the point r4 at which the arrival time T1 is t11 and the outflow time T2 is t2max is displayed in the region R4. A color corresponding to the point r5 at which the arrival time T1 is t12 and the outflow time T2 is t2max is displayed in the region R5. No color is displayed in the region R6 (in FIG. 8, the region R6 is displayed in black corresponding to the background of the displayer 6).

In the present embodiment, the parametric image PI comprised of the colors corresponding to the arrival time T1 and the outflow time T2 can be displayed. It is therefore possible to easily perform a diagnosis based on the arrival time T1 and the outflow time T2. That is, according to the present embodiment, an image useful in diagnosis can be displayed.

A modification of the first embodiment will next be explained. In the modification, the arrival time T1 may be a time from the time at which a contrast agent has reached a predetermined region (will be called "reference region BR") in a contrast image to the time when the brightness of the contrast image exceeds the first threshold value Bth1, assuming that the time at which the contrast agent has reached the predetermined region is zero. The reference region BR is, for example, a portion in a region in which a contrast image CI is being displayed at a subject, at which the contrast agent arrives first. The time at which the contrast agent has reached the reference region BR may be, for example, a time at which the brightness of the reference region BR exceeds the first threshold value Bth1.

The reference region BR may be set by an operator using the operation unit 7.

### (Second Embodiment)

A second embodiment will next be described. The description of the same items as in the first embodiment will however be omitted.

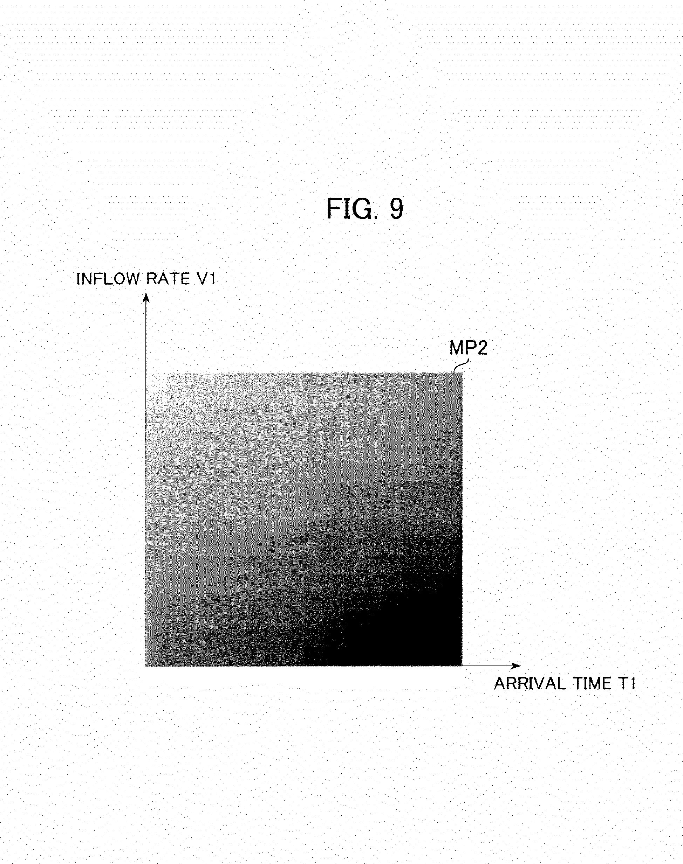

In the present embodiment, the display controller 5 generates a parametric image PI using a two-dimensional map MP2 shown in FIG. 9. In the two-dimensional map MP2, the horizontal axis indicates the arrival time T1, and the vertical axis indicates the rate V1 of inflow of a contrast agent at a part at which the contrast agent has arrived.

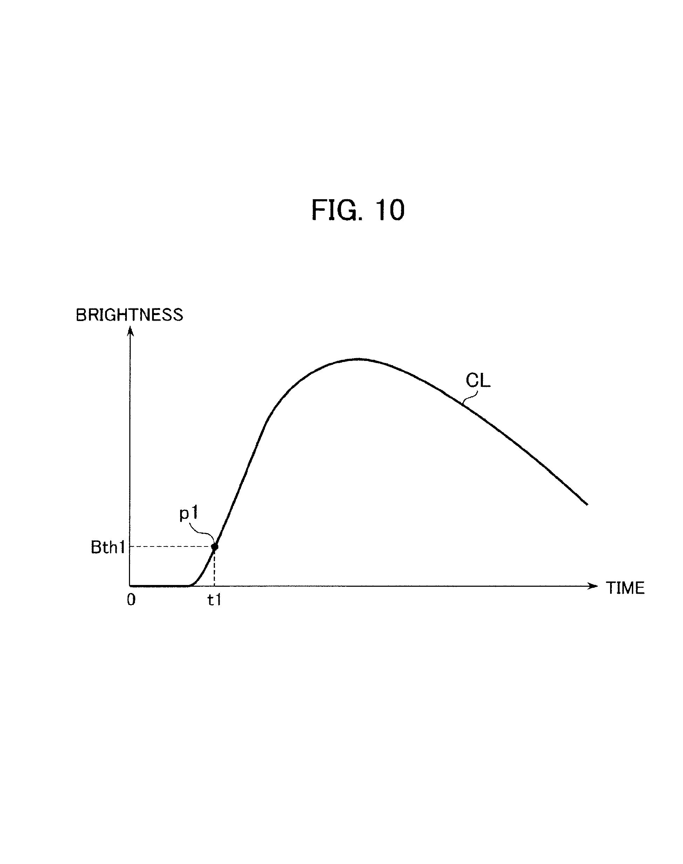

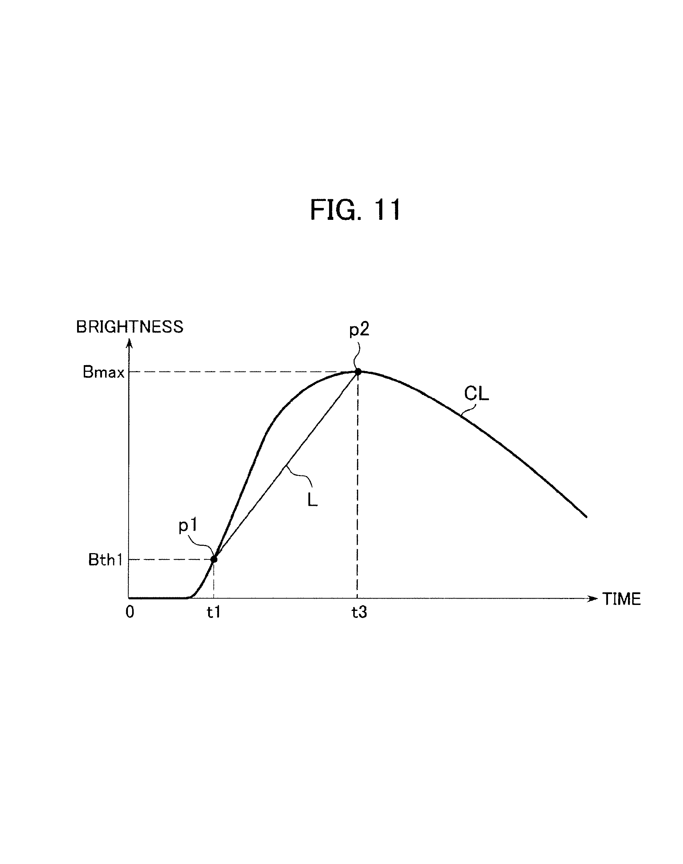

The inflow rate V1 indicates the velocity of a rise in the brightness in a contrast image CI due to the contrast agent at the part at which the contrast agent has arrived. Assuming that a point of a time t1 at which the brightness has reached the first threshold value Bth1 is p1 at a brightness change curve CL as shown in FIG. 10, for example, the gradient of the tangent to the curve at the point p1 may be set as the inflow rate V1. Alternatively, assuming that a point of a time t3 at which the brightness has reached maximum brightness value Bmax is p2 at a brightness change curve CL as shown in FIG. 11, the slope of a line segment L that connects the point p2 and the point p1 to each other, and the length of time from the times t1 to t3 may be set as the inflow rate V1.

The display controller 5 may calculate the inflow rate V1, based on contrast-mode data or may calculate the inflow rate V1, based on contrast image data. The inflow rate V1 is one example illustrative of an embodiment of a parameter indicative of the state of inflow of the contrast agent.

In the present embodiment, a parametric image PI comprised of colors corresponding to the arrival time T1 and the inflow rate V1 can be displayed. It is thus possible to display an image useful in diagnosis.

### (Third Embodiment)

A third embodiment will next be described. The description of the same items as those in the first and second embodiments will however be omitted.

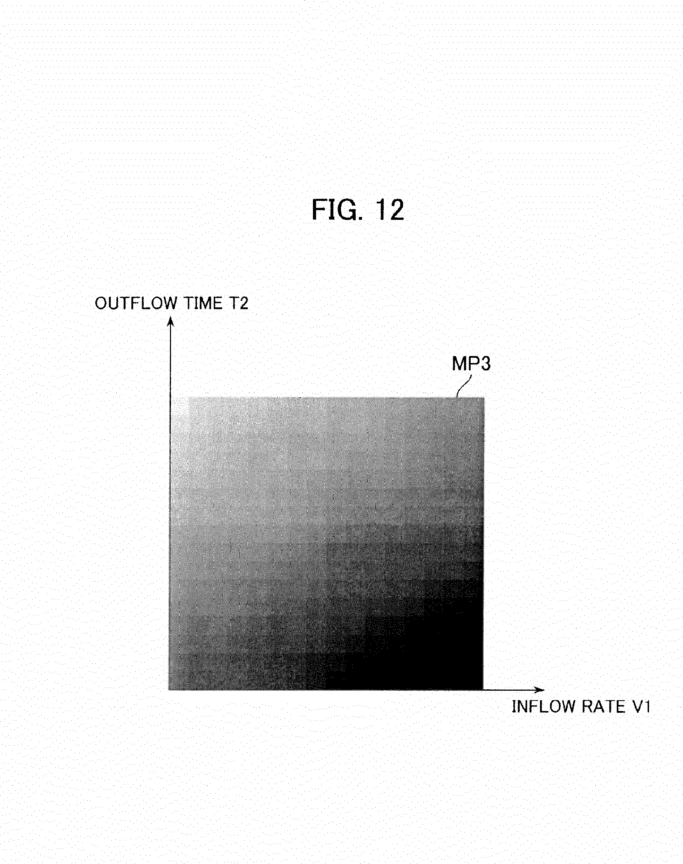

In the present embodiment, the display controller 5 generates a parametric image PI using a two-dimensional map MP3 shown in FIG. 12. In the two-dimensional map MP3, the horizontal axis indicates the inflow rate V1, and the vertical axis indicates the outflow time T2.

In the present embodiment, a parametric image PI comprised of colors corresponding to the inflow rate V1 and the outflow time T2 can be displayed. It is thus possible to display an image useful in diagnosis.

### (Fourth Embodiment)

A fourth embodiment will next be explained. The description of the same items as those in the first, second and third embodiments will however be omitted.

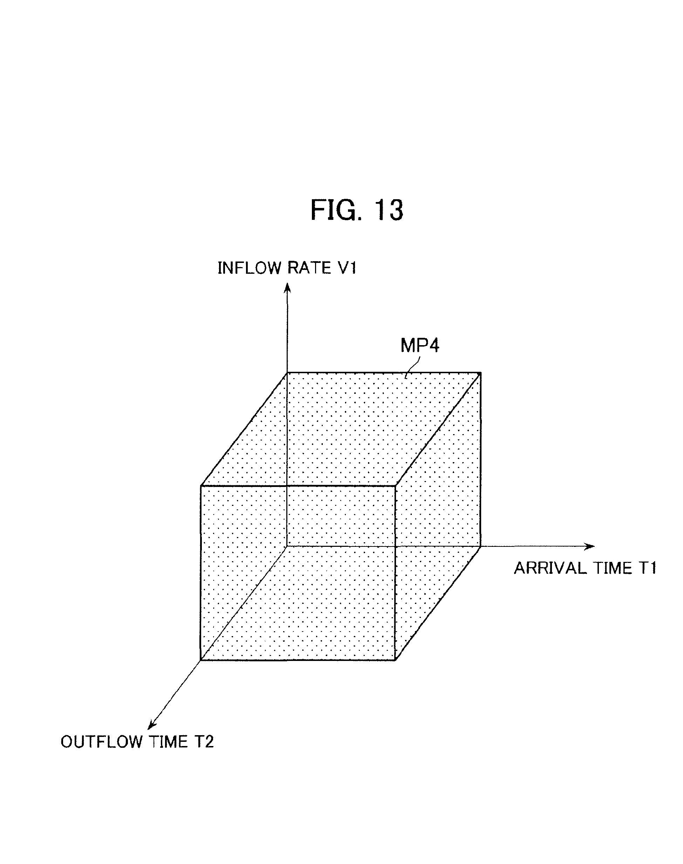

In the present embodiment, the display controller 5 generates a parametric image PI using a three-dimensional map MP4 shown in FIG. 13. The three-dimensional map MP4 is a color map comprised of three axes of the arrival time T1, the outflow time T2 and the inflow rate V1. In FIG. 13, however, the three-dimensional map MP4 is simply illustrated in a cube.

In the present embodiment, a parametric image PI comprised of colors corresponding to the arrival time T1, the outflow time T2 and the inflow rate V1 can be displayed. It is thus possible to display an image useful in diagnosis.

Although the present invention has been explained by the respective embodiments as described above, it is needless to say that the present invention can be changed in various ways within the scope that does not change the gist of the invention. For example, the parameter indicative of the state of inflow of the contrast agent and the parameter indicative of the state of outflow of the contrast agent are an example and not limited to the above.

The time taken as the starting point of the outflow time T2 is not limited to the time t1. For example, the outflow time T2 may be the time of zero at which the input to notify the administration of the contrast agent is performed or a time from the time at which the contrast agent has reached the reference region BR to the time t2 (refer to FIG. 4).

### [Explanation of Reference Numerals]

- 1: Ultrasonic Diagnostic Apparatus
- 5: Display controller
- 42: Contrast-mode data processor (contrast-mode signal processor)
- PI: Parametric image
- MP1: through MP3 Two-dimensional maps
- MP4: Three-dimensional map

## Claims

1. An ultrasonic diagnostic apparatus (1) comprising:
a display controller (5) which causes an image generated using a map taking as axes, two or more parameters of parameters each indicative of a state of inflow of a contrast agent administered to a subject and parameters each indicative of a state of outflow of the contrast agent to be displayed based on the two or more parameters.

2. The ultrasonic diagnostic apparatus (1) according to claim 1, wherein the parameters each indicative of the state of inflow of the contrast agent include an arrival time taken until the contrast agent arrives, and a rate of inflow of the contrast agent at a portion at which the contrast agent has arrived.

3. The ultrasonic diagnostic apparatus (1) according to claim 1 or 2, wherein the parameters each indicative of the outflow state of the contrast agent include an outflow time taken until the contrast agent flows out from a portion in which the contrast agent has flowed.

4. The ultrasonic diagnostic apparatus (1) according to any one of claims 1 to 3, wherein the display controller (5) causes the image generated based on the map to be displayed on a contrast image of the subject.

5. The ultrasonic diagnostic apparatus (1) according to any one of claims 1 to 4, wherein each of the parameters is specified based on data of the contrast image of the subject.

6. The ultrasonic diagnostic apparatus (1) according to claim 5, including a contrast-mode signal processing unit which performs signal processing for generating a contrast image on each of echo signals obtained by transmitting ultrasound for generating the contrast image,
wherein each of the parameters is specified based on data obtained by the contrast-mode signal processing unit.

7. The ultrasonic diagnostic apparatus (1) according to claim 6, wherein each of the parameters is specified based on raw data prior to scan conversion by a scan converter, which corresponds to the data obtained by the contrast-mode signal processing unit.

8. The ultrasonic diagnostic apparatus (1) according to claim 6 or claim 7, wherein each of the parameters is specified based on contrast image data obtained after the scan conversion of the data obtained from the contrast-mode signal processing unit by the scan converter.

9. A control program for an ultrasonic diagnostic apparatus (1), which causes a computer to execute:
a display control function which causes an image generated using a map taking as axes, two or more parameters of parameters each indicative of a state of inflow of a contrast agent administered to a subject and parameters each indicative of a state of outflow of the contrast agent to be displayed based on the two or more parameters.
